# EUROPÄISCHE PATENTSCHRIFT

(11) **EP 2 364 670 B1**
(45) Veröffentlichungstag und Bekanntmachung des Hinweises auf die Patenterteilung: **05.09.2018**
(21) Anmeldenummer: 11169560.7
(22) Anmeldetag: 20.07.2005
(51) Int. Cl.: A61F 2/06, D05B 3/00, D05B 23/00, D05B 21/00, D05B 39/00

(54) **Vorrichtung zur Erzeugung einer Krümmung in einer Gefässprothese**
Device for producing a curvature in a vascular prosthetis
Dispositif pour la production d'une courbure dans une prothèse de vaisseau

(30) Priorität: 12.08.2004 DE 102004039980; 21.07.2004 DE 102004035272
(43) Veröffentlichungstag der Anmeldung: 14.09.2011
(62) Teilanmeldung aus: 05766106.8
(73) Patentinhaber: AESCULAP AG, 78532 Tuttlingen (DE); Sievers, Hans-Hinrich, Prof. Dr. med., 24119 Kronshagen (DE)
(72) Erfinder: Sievers, Hans-Hinrich, 24119 Kronshagen (DE); Lippoth, Lisa, 78588 Denkingen (DE); Merckle, Christof, 68199 Mannheim (DE); Goldmann, Helmut, 78532 Tuttlingen (DE); Scherberich, Jürgen, 47906 Kempen (DE)
(74) Vertreter: Patentanwälte Ruff, Wilhelm, Beier, Dauster & Partner mbB

(56) Entgegenhaltungen:
- EP-A- 0 251 331
- EP-A- 1 178 144
- DE-A1- 10 242 153
- US-A- 2 836 181
- US-A- 4 169 422
- US-A- 4 544 599
- US-A- 4 730 566

## Beschreibung

Die Erfindung betrifft die Verwendung einer Kettenstichnähmaschine zur Herstellung einer textilen Gefäßprothese mit einer umlaufenden, von Falten gebildeten Plissierung und einer mindestens über einen Teilabschnitt ausgebildeten bogenförmigen Längsbiegung durch asymmetrische verkürzende Raffung der Prothesenwand durch mindestens eine längs des Teilabschnittes verlaufende Längsnaht. gemäss Anspruch 1. Die Prothese dient insbesondere zum Ersatz des Aortenbogens und Teilen der Aorta aszendenz und deszendenz.
Aus der WO 03/034948 A1 ist eine plissierte Gefäßprothese bekannt, bei der eine Bogenform dadurch stabilisiert wird, dass jeweils zwei benachbarte Faltenberge der Plissierung durch querverlaufende Nähte miteinander vernäht werden, wobei sich die querverlaufenden Nähte in etwa über den halben Durchmesser der Gefäßprothese in Querrichtung erstrecken. Es ist unter anderem aus dieser Druckschrift auch bekannt, die Bogenform von Prothesen durch im Bereich des Bogens eingelegte Stents zu stabilisieren. In der WO 03/051232 A1 sind zur Stabilisierung des Bogens von Gefäßprothesen verschiedene Kombinationen von Längs- und/oder Quernähten beschrieben. Die Ergebnisse sind zufriedenstellend. Eine einfachere Herstellungsweise, insbesondere eine maschinelle Ausbildung der Bogenstabilisierung ist jedoch erwünscht.
In einer vorveröffentlichten älteren Patentanmeldung DE 103 28 175.4 wird unter anderem vorgeschlagen, die Plissierungsfalten in radialer oder tangentialer Richtung zusammenzudrücken, um maschinelle Nähte in einfacher Weise ausbilden zu können. Hierbei wird eine Verformung der Gefäßprothese im Querschnitt in Kauf genommen.
Aus der EP 1 178 144 A1 ist eine Nähmaschine bekannt, in welcher ein zylindrischer Rahmen zum Halten von schlauchförmigen Objekten integral an einem zylindrischen Antriebsring angebracht ist, der hin- und hergehend in einer Drehrichtung um einen Freiarm herum und entlang einer Achse des Freiarms linear hin- und hergehend angetrieben wird. Eine Kettenstichnähmaschine wird zum Beispiel in US4169422 offenbart. Der Erfindung liegt die Aufgabe zugrunde, auf einfache Weise eine Stabilisierung des Bogens von Gefäßprothesen auszubilden, die insbesondere auch eine einfache maschinelle Fertigung mit verbessertem Ergebnis erlaubt.
Die Erfindung ist dadurch gekennzeichnet, dass die mindestens eine Längsnaht die Prothesenwand unter Erhalt der faltenbalgartigen Struktur und in in Längsrichtung kompaktiertem Zustand der Plissierung rafft.
Durch die Erfindung ergeben sich zahlreiche Vorteile. Einerseits wird durch die Kompaktierung der Plissierung und durch das damit zusammenhängende dichte Aneinanderliegen der einzelnen Falten der Plissierung eine effektive Ausgestaltung des Bogens erhalten. Auf der anderen Seite stützen sich die eng aneinander liegenden Falten der Plissierung gegenseitig ab, so dass die Anbringung einer Naht, deren Stiche die kompaktierte Prothesenwand radial durchdringen in einfacher Weise angebracht werden, ohne dass die Struktur der Plissierung aufgehoben, d.h. die Plissierung in radialer Richtung zusammengedrückt wird. Durch die Kompaktierung kann die Naht auch stramm angelegt und damit zugfest ausgebildet werden. Der oder die Fäden der mindestens einen Naht können die Falten des kompaktierten Faltenbalges umschlingen, wodurch der kompaktierte Zustand besonders gut aufrecht erhalten werden kann.
Die Gefäßprothese kann in üblicher Weise gewebt oder gewirkt sein. Die Plissierung kann mit in sich geschlossenen Ringen ausgebildet sein oder auch wendelförmig verlaufen. Die Faltentiefe der Plissierung liegt in der Regel bei 0,5 bis 2,5 mm, vorzugsweise 1 bis 1,5 mm. Es sind in der Regel 3 bis 12, insbesondere 4 bis 8 Falten pro cm Prothesenlänge im entspannten nicht kompaktierten Zustand (gerades Stück) vorhanden. Die Bogenform der Gefäßprothese kann durch thermische Fixierung vorgeformt sein, was sich günstig auswirkt.
Die Raffung kann ausschließlich durch die mindestens eine Längsnaht vorgenommen sein, gegebenenfalls unterstützt durch die thermische Vorfixierung. Es sind keine Quernähte oder weitere die Bogenform unterstützende Einrichtungen, wie Gestelle oder Stents vorgesehen.
Die mindestens eine Längsnaht kann weiterhin von mindestens einem durchgehenden Faden gebildet sein. Diese Ausbildung erlaubt eine besonders einfache Herstellung. Wie bereits erwähnt, ist die mindestens eine Naht als Maschinennaht ausgebildet. Sie wird insbesondere mit Hilfe einer Langarmnähmaschine gefertigt. Die mindestens eine Naht ist ferner auf einer durch Stauchung kompaktierten Gefäßprothese mit eng aneinander liegenden Falten der Plissierung ausgebildet und kann die Gefäßprothese an der Bogeninnenseite in diesem Zustand festhalten. Auf der Bogenaußenseite, die frei von einer Naht ist, ist die Plissierung unter Ausbildung des Bogens wieder aufgegangen.

Die Naht ist ein Kettenstich. Weiterhin können mehrere Nähte, insbesondere 2 bis 4 Nähte, in Längsrichtung parallel nebeneinander verlaufen. Dies kann insbesondere bei Aortenbögen mit großem Durchmesser von Vorteil sein. Der Abstand zwischen den parallelen Längsnähten kann mit Vorteil im Bereich von 1 bis 15, insbesondere 1 bis 3 mm, liegen, wobei Abstände über 10 mm bis 15 mm in der Regel nur bei zwei, außermittigen Längsnähten in Frage kommen. Es ist auch möglich, Teilnähte vorzusehen, die sich gegenseitig ergänzen. So können in Längsrichtung mehrere parallele Teilnähte verlaufen, die sich vorzugsweise gegenseitig überlappen. Dies kann von Vorteil sein, wenn aus einem größeren Bogen Teilstücke ausgeschnitten werden. Es können auch mehrere Teilnähte mit Zwischenabständen in Längsrichtung hintereinander angeordnet sein. Dies bietet die Möglichkeit, den Bogen zu erweitern, d.h. den Radius zu vergrößern bei gleichzeitiger Aufrechterhaltung des kompaktierten Zustandes der Plissierung im Nahtbereich. Auch die Nahtenden der Teilnähte sind vorzugsweise gegen ein unerwünschtes Lösen gesichert. Eine Sicherung der Nahtenden kann beispielsweise durch Nahtverdichtung, Vernähen, Verknoten oder Verriegeln vorgenommen werden. Weiterhin ist es mit Vorteil möglich, mehrere Nähte, insbesondere Teilnähte, mit dem selben Faden bzw. den selben Fäden ohne Fadenunterbrechung auszubilden. Die nahtfreien Zwischenstücke der Prothese können dann vom durchgehenden Faden überbrückt werden. Sämtliche Längsnähte sind vorzugsweise nur im Bereich der Bogeninnenseite angeordnet.

Die Länge des gerafften Längsbereiches der Gefäßprothese kann in Abhängigkeit vom Bogenmaß und/oder Durchmesser der Gefäßprothese mindestens 10 mm, insbesondere 20 bis 70 mm, betragen. Das Bogenmaß kann zwischen 60° und 270°, insbesondere zwischen 90° und 180°, liegen und erforderlichenfalls auch größer sein. Der lichte Durchmesser liegt in der Regel bei 8 mm bis 50 mm, bei Aortenbögen eher im oberen Bereich. Die Nahtlänge von Teilnähten, insbesondere von hintereinander und/oder versetzt nebeneinander angeordneten Teilnähten liegt in der Regel bei mindestens 5 mm, insbesondere im Bereich von 10 bis 20 mm. Die Stichlänge, insbesondere Schlingenlänge, der mindestens einen Naht beträgt mit Vorteil 1 bis 3 mm, insbesondere ca. 2 mm. Pro Stichlänge bzw. Schlingenlänge der Naht können zwei bis vier dicht aneinander liegende Plissierungsfalten, insbesondere drei Plissierungsfalten umfasst, insbesondere vollständig umschlungen sein. Da die Stiche nicht genau mit den Plissierfalten zusammenzufallen brauchen, braucht die Zahl der Plissierungsfalten, die von einer Stichlänge umfasst werden, nicht ganzzahlig zu sein.

Die mindestens eine Längsnaht kann mit Vorteil zwischen zwei in Längsrichtung der Gefäßprothese verlaufenden Guidelines verlaufen. Dadurch sind die Guidelines auf beiden Seiten des Bogens gut zu erkennen.

Mit Vorteil heben sich die Fäden in der Färbung deutlich von der Farbe des textilen Grundmaterials der Gefäßprothese ab. Dadurch ist auch während der Operation der Verlauf der Bogeninnenseite zu erkennen.

Als Fäden werden vorzugsweise multifile Fäden verwendet, insbesondere beschichtete Fäden. Es können Fäden mit der Fadenstärke 1/0 bis 5/0, vorzugsweise 2/0 bis 4/0 verwendet werden. Bei asymmetrischen Nähten, die auf der einen Seite der Gefäßwandung ein anderes Erscheinungsbild zeigen als auf der anderen Seite, ist es mit Vorteil möglich, die mindestens eine Naht im umgestülpten Zustand der Gefäßprothese anzubringen und danach zurückzustülpen.

Es wird auch ein Verfahren zur Erzeugung einer gezielten, bleibenden Krümmung an einer künstlichen Gefäßprothese offenbart. Das Verfahren ist dadurch gekennzeichnet, dass eine Gefäßprothese, die gegebenenfalls einen thermisch vorfixierten Bogen aufweist, axial gestaucht bzw. kompaktiert wird und die Naht an der Stelle bzw. den Stellen, an denen eine elastische Ausdehnung verhindert werden soll, angebracht wird, wonach man die Prothese unter Bildung der Bogenform axial expandieren lässt.

Die Erfindung umfasst eine Vorrichtung zur Erzeugung einer gezielten, bleibenden Krümmung an einer künstlichen Gefäßprothese, bestehend aus einer Kettenstichnähmaschine mit einem Arm, der den Nähnadelantrieb für eine Nähnadel umfasst, einer Spule mit einem Faden, dessen Ende mit der Nähnadel geführt werden kann, einem Träger für das zu nähende Material mit einem Greifer, der den Faden nach Durchstehen des Materials mit der Nadel verkettet, und einer Vorschubeinrichtung für das Material.

Die gestauchte Prothese wird von einem Stützrohr aufgenommen, auf dem auch die Stauchung vorgenommen werden kann.

Dadurch, dass die Gefäßprothese auf ein Stützrohr aufgebracht und auf diesem gestaucht wird, ist der Nähvorgang besonders einfach. Die Gefäßprothese wird über ihre Länge sehr gleichmäßig gestaucht. Das Stützrohr verhindert eine Biegung der Gefäßprothese während des Nähvorgangs. Der Faden, der in axialer Richtung in die Wandung des elastischen Rohres eingenäht wird, verhindert nach Abziehen der Gefäßprothese vom Stützrohr, dass die Gefäßprothese auf einem Teil des Umfangs, nämlich im Bereich des genähten Bereichs zurückfedern kann. Auf der gegenüberliegenden Seite der Naht wird die Gefäßprothese weniger stark behindert zurückzufedern. Auf diese Weise nimmt die Gefäßprothese nach Abziehen von dem Stützrohr automatisch einen Bogen an, dessen Krümmung von der ursprünglichen Stauchung und der Länge der Naht abhängig ist.

Es ist ein Vorteil, wenn das Stützrohr während des Nähvorgangs in axialer Richtung bewegt wird. Dies ermöglicht eine gleichförmige Naht.

Es ist vorteilhaft, die Naht mittels einer Nähnadel zu erzeugen. In diesem Fall ist der einfache Kettenstich möglich und empfehlenswert.

Vorzugsweise taucht die Nähnadel durch die Gefäßprothese und durch ein in die Wandung des Stützrohres eingebrachtes Langloch oder eingebrachten Schlitz in das Innere des Stützrohres ein. Dann kann der Faden innerhalb der Gefäßprothese verknotet werden.

Aus dem gleichen Grund ist es vorteilhaft, wenn ein Fadengreifer im Inneren der Gefäßprothese und insbesondere des Stützrohres angebracht ist, der den Faden von der Nadel abnimmt und verkettet.

Um die Geschwindigkeit des Verfahrens zu erhöhen, ist es sinnvoll, den Fadengreifer rotieren zu lassen.

Es erhöht die Sicherheit, wenn das Stützrohr während seiner axialen Bewegung den rotierenden Fadengreifer umschließt. Es werden dadurch Verletzungen durch Hineingreifen vermieden.

Es ist von ganz besonderem Vorteil, wenn die Gefäßprothese vor der Aufnahme auf das Stützrohr auf links gewendet wird, also die Innenoberfläche nach außen und umgekehrt. Das bewirkt nämlich, dass die im Kettenstichverfahren innen vorgenommene Verknotung anschließend wieder nach außen gewendet wird. Damit stellen die Knoten kein Hindernis für die Blutzirkulation dar. Das so angewendete Verfahren erlaubt zudem eine genauere Kontrolle der Nähnaht und eine bessere Bearbeitungsmöglichkeit der Verriegelungen oder Verknotungen am Anfang und Ende der Naht. Beispielsweise kann das Kürzen der Endfäden wesentlich genauer durchgeführt werden.

Es ist vorteilhaft, wenn die Stauchung der Gefäßprothese auf dem Stützrohr manuell vorgenommen wird. Das ermöglicht die einfachste und schnellste Anwendung des Verfahrens.

Bei absoluten Präzisionsarbeiten oder mehrfach wiederkehrenden gleichen Arbeitsvorgängen kann es auch sinnvoll sein, eine Stauchungsvorrichtung für die Stauchung einzusetzen.

Dazu ist es sinnvoll, die Gefäßprothese wenigstens an einem verstellbaren Endanschlag abzustützen. Damit ist eine genaue Einstellmöglichkeit und Replizierbarkeit des Vorgangs gewährleistet.

Für die Vorrichtung zur Herstellung einer gezielten bleibenden Krümmung einer Gefäßprothese ist es vorteilhaft, wenn ein Stützrohr vorhanden ist, auf das die Gefäßprothese in gestauchter Form geschoben werden kann. Für den anschließenden Nähvorgang ist dadurch ein sicherer Halt gewährleistet.

Vorzugsweise entspricht der Außendurchmesser des Stützrohres in etwa dem Innendurchmesser der Gefäßprothese. Dadurch wird der sichere Halt unterstützt.

Es ist von Vorteil, wenn das Stützrohr mit einer Vorschubeinrichtung verbunden ist. Dann wird die Gefäßprothese in gestauchter Form mit festem Sitz auf dem Stützrohr unterhalb der Nadel geführt, was zu einem gleichmäßigen Nahtbild führt.

Weitere vorteilhafte Ausgestaltungen der Vorrichtung sind in den Unteransprüchen beschrieben.

Weitere Merkmale und Vorteile der Erfindung ergeben sich aus der nachfolgenden Beschreibung von bevorzugten Ausführungsformen in Verbindung mit der Zeichnung und den Unteransprüchen. Hierbei können die einzelnen Merkmale jeweils für sich alleine oder in Kombination miteinander verwirklicht sein. In der Zeichnung zeigen:
- Figur 1:: eine Seitenansicht einer Ausführungsform eines Aortenbogens,
- Figur 2:: einen Schnitt entlang der Linien II.-II. nach Figur 1,
- Figur 3:: eine schematische Darstellung des Bereichs A an der Innenseite des Aortenbogens nach Figur 1,
- Figur 4:: eine schematische Darstellung des Bereichs A in einer anderenVariante,
- Figur 5:: eine schematische Darstellung des Bereichs A in einer weiterenVariante,
- Figur 6:: eine schematische Darstellung des Bereichs A in einer weiterenVariante,
- Fig. 7:: schematisch eine hergestellte Gefäßprothese in perspektivischer Darstellung,
- Fig. 8:: schematisch eine erfindungsgemäße Vorrichtung zur Herstellung der gekrümmten Gefäßprothese.

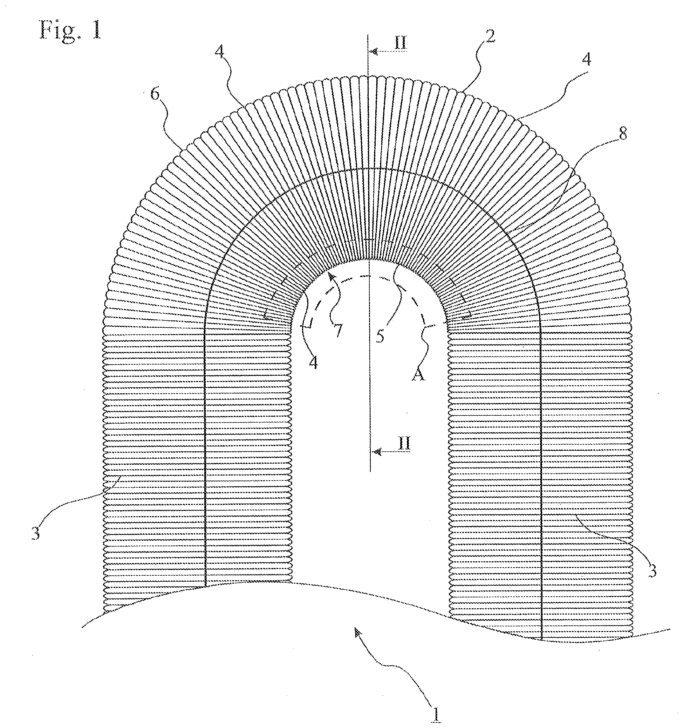

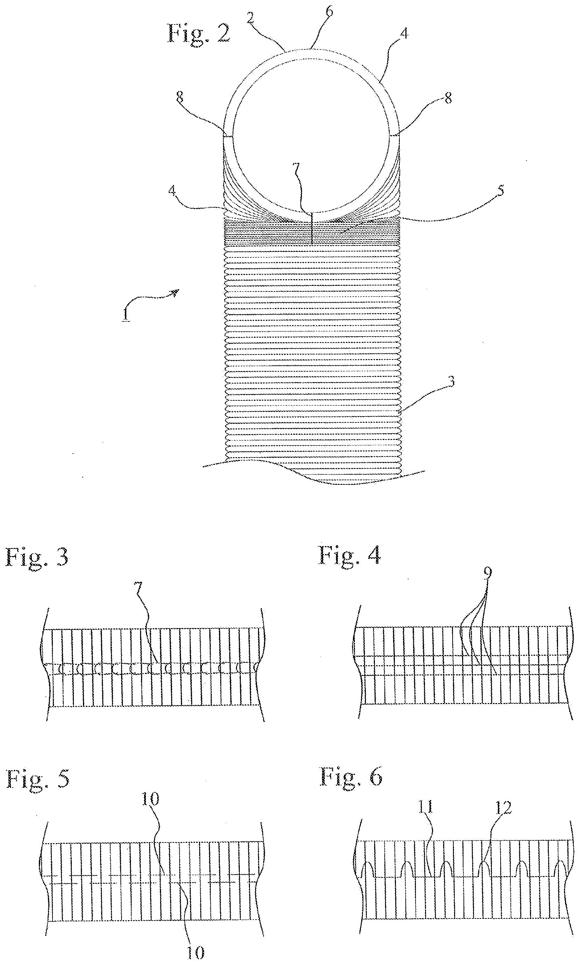

Bei der in der Zeichnung dargestellten Variante gemäß den Figuren 1 und 2 ist ein Aortenbogen 1 vorgesehen, der einen Bogenabschnitt 2 mit einer Umbiegung von 180° aufweist. An den Bogenabschnitt schließen sich gerade Endstücke 3 an, die zum Annähen an die verbleibenden Reste der natürlichen Aorta dienen. Der Aortenbogen 1 besitzt einen Innendurchmesser von 33 mm sowie eine Plissierung mit sieben Falten 4 pro cm. Die Faltentiefe beträgt ca. 1,5 mm. Die Falten 4 sind an der Bogeninnenseite 5 dicht aneinander liegend und erweitern sich stetig bis zur Bogenaußenseite 6. Der verdichtete kompakte Zustand der Falten an der Bogeninnenseite 5 wird durch eine einzige Längsnaht 7 stabilisiert. Die Stiche der Längsnaht durchdringen den kompaktierten Faltenbalg an der Bogeninnenseite in radialer Richtung und bilden zusammenhängende schlaufenartige Schlingen, die jeweils ca. drei Falten umschließen. Auf den beiden Seiten des Aortenbogens befindet sich je eine Guideline 8, wobei die beiden Guidelines verschieden breit ausgebildet sind. Das Nahtmaterial der Längsnaht 7 besitzt eine Färbung, die sich von der weißen Grundfärbung des Aortenbogens auch im mit Blut getränktem Zustand deutlich abhebt.

Der Faden der Längsnaht 7 ist ein multifiler mit Silikon beschichteter Faden mit der Fadenstärke 3/0. Die Naht beruht auf einem Einfadensystem, bei der ein Faden im Einfachkettenstich (Fig. 3) vernäht ist. Bei diesem System wird eine Fadenschlaufe radial durch die Prothesenwand geführt, in Nahtrichtung umgelegt und beim nächsten Stich durch die nächste Schlaufe erfasst. Dadurch bildet sich eine Längskette von zusammenhängenden Schlaufen. Auf der anderen Seite ist der einzelne Faden von Stich zu Stich in Längsrichtung geführt.

Zur Herstellung der Längsnaht wird zweckmäßigerweise ein zur Bildung des Aortenbogens verwendeter Abschnitt einer gewirkten Gefäßprothese mit Plissierung bzw. Faltenbalg auf einer Stützvorrichtung geradlinig in Längsrichtung so weit wie möglich zusammengeschoben, bis die einzelnen Falten des Faltenbalges in kompaktiertem Zustand parallel dicht nebeneinander liegen. In diesem Zustand wird die Längsnaht zum Beispiel mit einer Langarmnähmaschine angefertigt, wobei der Faden die kompaktierten Falten radial durchdringt. Die Fadenenden werden durch Fadenverdichtung gegen unerwünschtes Aufgehen verriegelt. Es sind auch Zwischenverriegelungen möglich. Beim Abziehen des kompaktierten Faltenbalges von der Stützvorrichtung entspannen sich die Falten der Plissierung wieder bis auf die Falten, die durch die Längsnaht an der Bogeninnenseite an einem Auseinandergehen gehindert sind. Dadurch bildet sich ein gleichmäßiger Aortenbogen aus. Die Länge der Längsnaht beträgt ca. 50 mm.

Die Figuren 3 bis 6 zeigen verschiedene Varianten von Längsnähten, die sich ausnahmslos an der Bogeninnenseite befinden. Figur 3 zeigt die einzige Längsnaht 7 der Variante nach Figur 1. Bei der Variante nach Figur 4 sind drei parallele Längsnähte 9 vorgesehen, die einen Abstand von 1 bis 2 mm voneinander haben. Figur 5 zeigt Teillängsnähte 10, die in zwei Reihen im Abstand von 2 mm unter gegenseitigem Versatz angeordnet sind. Die Teillängsnähte haben eine Länge von ca. 10 mm. Die Fadenenden sind jeweils gegen Aufgehen gesichert. Figur 6 zeigt in einer Linie verlaufende Teillängsnähte 11, die jeweils Unterbrechungen 12 zwischen sich aufweisen. Die Unterbrechungen 12 haben die Folge, dass der Faltenbalg in deren Bereich nicht kompaktiert ist, d.h. nach der Anfertigung der Naht sich wieder ausdehnt. Durch solche Unterbrechungen ist es möglich, die Größe der Bogenweite bzw. des Bogenradius zu steuern. Die Teillängsnähte 11 und/oder die Unterbrechungen 12 zwischen diesen können durch entsprechende Steuerung der Naht auch unterschiedliche Größe aufweisen, wodurch von der Kreisbogenform abweichende Bogenformen ausgebildet werden können. Ist es bei Nähten, die auf der einen Seite ein anderes Erscheinungsbild haben als auf der anderen Seite erwünscht, eine bestimmte Nahtseite außen zu haben, dann kann dies dadurch beeinflusst werden, dass die Prothese vor dem Zusammenschieben und Anbringen der Naht gegebenenfalls umgestülpt und danach wieder zurückgestülpt wird.

Durch das Zusammenhalten der kompaktierten Bereiche des Faltenbalgs an der Bogeninnenseite wird eine gleichmäßige Bogenform erhalten. Der kreisrunde Innenquerschnitt des Aortenbogens bleibt trotz der maschinellen Fertigung der Naht, bei der ein gewisser Anpressdruck erforderlich ist, aufrecht erhalten.

Der Aortenbogen kann in üblicher Weise imprägniert und in steriler Form verpackt werden. Falls nicht die gesamte Bogenlänge zum Einsatz kommt, kann vor der Operation ein entsprechender Bogenabschnitt abgeschnitten werden. Hier sind Aortenbögen mit solchen Nähten bevorzugt, die Zwischenverriegelungen der Fäden aufweisen oder Teilnähte mit jeweiliger Endverriegelung besitzen.

Die maschinelle Fertigung kann auch noch dadurch erleichtert werden, dass mehrere Nähte, z.B. parallele Nähte oder Teilnähte, mit durchgehendem Faden genäht werden, der dann die Zwischenbereiche überbrückt, wie dies in Figur 6 schematisch dargestellt ist.

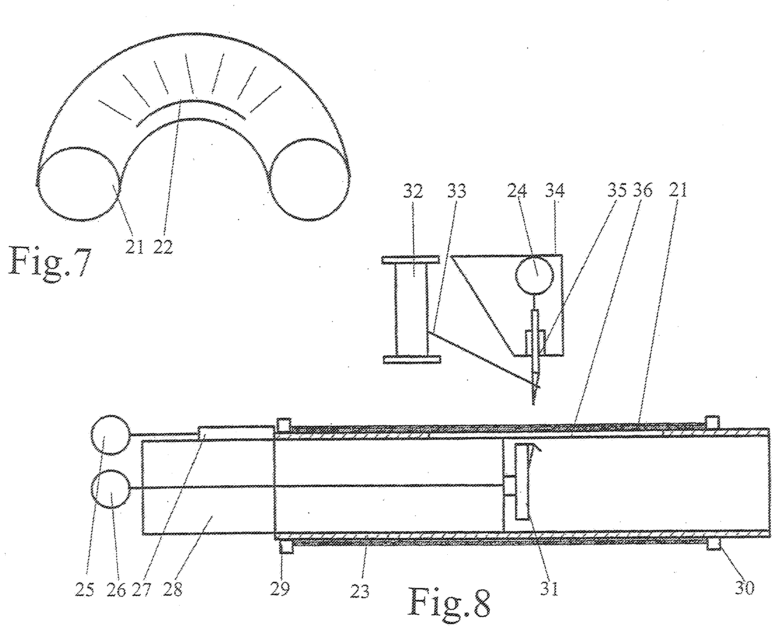

Fig. 7 stellt eine hergestellte elastische Gefäßprothese dar. Sie besteht aus einem biokompatiblen, dauerfesten Material, beispielsweise aus Polyethylenterephthalat. Die Größenordnung der Durchmesser bewegt sich im zweistelligen Millimeterbereich, vorzugsweise im Bereich von 20 bis 40 mm. Während der Herstellung der Krümmung wird die Gefäßprothese gestaucht auf ein Stützrohr 23 aufgeschoben und mit wenigstens einer, sich wenigstens über einen Teil der Wandungslänge erstreckenden Naht 22 versehen. Diese sorgt nach dem Abziehen der Gefäßprothese 21 vom Stützrohr 23 und nach der Entspannung der elastischen Stauchung dafür, dass die Stauchung im Bereich der Naht 22 erhalten bleibt. Mit größer werdendem Abstand von der Naht 22 auf dem Umfang der Gefäßprothese 21 entspannt sich die Wandung immer deutlicher und kann sich wieder elastisch ausdehnen. Dadurch kommt es automatisch zu einer Verbiegung der Gefäßprothese 21. Die Naht 22 wird mit einem biokompatiblen Faden 33, z.B. aus Polyethylenterephthalat erzeugt. Es ist manchmal vorteilhaft, die Gefäßprothese 21 vor dem gestauchten Aufbringen auf das Stützrohr 23 umzuwenden, das heißt, die Außenseiten nach innen zu kehren und umgekehrt. Wendet man die Gefäßprothese 21 nach Fertigstellung wieder, sind die Verknotungen dann außen und stören nicht die Blutzirkulation.

In Fig. 8 ist in vereinfachter Darstellung eine geeignete Vorrichtung zur Erzeugung einer Krümmung einer Gefäßprothese 21 dargestellt. Ihre Ausgestaltung ähnelt sehr einer bekannten Kettenstichnähmaschine mit einem unteren Träger 28 mit Fadengreifer 31 zur Materialauflage und einem Arm 34, von dem die Nadelbewegung eingeleitet wird.

In der erfindungsgemäßen Vorrichtung ist der feststehende Träger 28 von einem Stützrohr 23 umgeben. Mit Hilfe einer Vorschubeinrichtung 27, die mit dem Stützrohr 23 verbunden ist, kann das Stützrohr 23 axiale Bewegungen ausführen. Dafür ist ein Vorschubantrieb 25 vorgesehen.

Der Arm 34 unterscheidet sich nicht von dem einer handelsüblichen Kettenstichnähmaschine. In einer Führung wird der Hub der Nähnadel 35 durch einen Nähnadelantrieb 24 vorgenommen. Von einer in der Nähe angeordneten Spule 32 wird ein Faden 33 zur Nähnadel 35 geführt.

Das Stützrohr 23 ist mit einem Langloch 36 in der Wandung in axialer Ausrichtung versehen, durch das die Nähnadel 35 während des Nähvorgangs hindurchtreten kann. Die Länge des Langlochs 36 entspricht dem maximalen Vorschubweg der Vorschubvorrichtung.

Das Stützrohr 23 dient zur Aufnahme der Gefäßprothese 21. Diese kann entweder manuell auf dem Stützrohr 23 gestaucht werden. Dazu sind ein oder zwei verstellbare Anschläge für die Gefäßprothese 21 auf dem Stützrohr vorgesehen. Es ist aber auch möglich, den Vorgang zu automatisieren und einen nicht in der Figur dargestellten motorisch verfahrbaren Anschlag 30 vorzusehen, der bei jeder zu bearbeitenden Gefäßprothese 21 zu einer gleichmäßigen Stauchung führt.

Es ist häufig von Vorteil, wenn der Innendurchmesser der Gefäßprothese 21 dem Außendurchmesser des Stützrohres 23 in etwa entspricht, vorzugsweise nur geringfügig kleiner ist. Dadurch wird ein sicherer Halt auf dem Stützrohr 23 während des Nähvorgangs gewährleistet.

Durch den Vorschub des Stützrohres 23 kann die gestauchte Gefäßprothese 21 oberhalb des Langlochs 36 mit einer Naht 22 versehen werden. Am Ende des Trägers 28, innerhalb des Stützrohres 23 ist ein Fadengreifer 31 angeordnet, der den mit der Nähnadel 35 eingebrachten Faden 33 aufnimmt und verkettet. Wie bei bekannten Kettenstichnähmaschinen ist es von Vorteil, wenn der Fadengreifer 31 rotiert und die Rotation mit der Hubbewegung der Nadel abgestimmt ist. Dazu ist ein Greiferantrieb 26 vorgesehen.

Vorteilhafterweise umschließt das Stützrohr 23 während des gesamten Nähvorgangs den Fadengreifer 31. Die Gefahr, mit der Hand in den rotierenden hakenförmigen Fadengreifer 31 zu fassen, ist damit ausgeschlossen. Das Stützrohr 23 sollte dabei von seiner Steifigkeit her so ausgelegt sein, dass sich seine Querschnittsform während des Nähvorgangs aufgrund der radialen Belastung durch die Nähnadel 35 auf die aufgeschobene Gefäßprothese 21 nicht oder nur unmerklich verändert.

Es hat sich als vorteilhaft erwiesen, wenn der Durchmesser eines rotierenden Fadengreifers 31 nicht mehr als 3 mm kleiner ist als der Innendurchmesser des Stützrohres 23. Oder umgekehrt ausgedrückt braucht der Innendurchmesser des Stützrohres 23 - ausreichende Steifigkeit des Stützrohrmaterials vorausgesetzt - nur wenig größer zu sein als der Greiferdurchmesser. Dies ist deshalb von Vorteil, weil die Fadengreifer 31 einen Mindestdurchmesser für das sichere Funktionieren beim Greifen und Verketten des Fadens 33 benötigen.

Es sei noch erwähnt, dass der Schutzumfang des Verfahrens auch umfassen soll, eine bereits auf thermischem oder chemischem Wege erzeugte Vorkrümmung einer Gefäßprothese zu stabilisieren. Ferner umfasst das Verfahren den Einsatz einer Gefäßprothese , die eine Plissierung aufweist. Eine solche Gefäßprothese lässt sich besonders gleichmäßig kompaktieren bzw. stauchen.

## Patentansprüche

1. Verwendung einer Vorrichtung bestehend aus einer Kettenstichnähmaschine mit
- einem Arm, der den Nähnadelantrieb (24) für eine Nähnadel (35) umfasst,
- einer Spule (32) mit einem Faden (33), dessen Ende mit der Nähnadel (35) geführt werden kann,
- einem Träger (28) für das zu nähende Material mit einem Fadengreifer (31), der den Faden (33) nach Durchstehen des Materials mit der Nadel verkettet, und
- einer Vorschubeinrichtung (27) für das Material, **gekennzeichnet dadurch, dass** die Vorrichtung ein Stützrohr (23) umfasst, über das die Gefäßprothese in gestauchter Form geschoben werden kann, zur Erzeugung einer gezielten, bleibenden Krümmung einer künstlichen Gefäßprothese, wobei zur Erzeugung der Krümmung mindestens eine Längsnaht die Prothesenwand unter Erhalt einer faltenbalgartigen Struktur und in in Längsrichtung kompaktiertem Zustand der Plissierung rafft.

2. Verwendung nach Anspruch 1, **dadurch gekennzeichnet, dass** der Außendurchmesser des Stützrohres (23) in etwa dem Innendurchmesser der Gefäßprothese (21) entspricht.

3. Verwendung nach Anspruch 1 oder 2, **dadurch gekennzeichnet, dass** das Stützrohr (23) mit der Vorschubeinrichtung (27) verbunden ist.

4. Verwendung nach einem der vorhergehenden Ansprüche, **dadurch gekennzeichnet, dass** das Stützrohr (23) eine so hohe Steifigkeit besitzt, dass sich seine Querschnittsform während des Nähvorgangs aufgrund der radialen Belastung durch die Nähnadel (35) auf die aufgeschobene Gefäßprothese (21) nicht oder nur unmerklich verändert.

5. Verwendung nach einem der vorhergehenden Ansprüche, **dadurch gekennzeichnet, dass** das Stützrohr (23) einen Fadengreifer (31) umschließt.

6. Verwendung nach Anspruch 5, **dadurch gekennzeichnet, dass** der Fadengreifer (31) rotiert.

7. Verwendung nach einem der Ansprüche 5 oder 6, **dadurch gekennzeichnet, dass** der Durchmesser des Fadengreifers (31) maximal 3 mm kleiner ist als der Innendurchmesser des Stützrohres (23).

8. Verwendung nach einem der vorhergehenden Ansprüche, **dadurch gekennzeichnet, dass** das Stützrohr (23) in der Wandung ein Langloch (36) aufweist, durch das die Nadel (35) während des Nähvorgangs hindurchtreten kann.

9. Verwendung nach einem der vorhergehenden Ansprüche, **dadurch gekennzeichnet, dass** das Stützrohr (23) auf seinem Außenumfang wenigstens einen axial verstellbaren Anschlag (29) für die Gefäßprothese (21) aufweist.

10. Verwendung nach einem der vorhergehenden Ansprüche, **dadurch gekennzeichnet, dass** das Stützrohr (23) auf seinem Außenumfang wenigstens einen weiteren Anschlag (30) zum Einspannen der gestauchten Gefäßprothese (21) aufweist.

## Claims

1. Use of a device composed of a chain-stitch sewing machine with
- an arm which includes the sewing needle drive (24) for a sewing needle (35),
- a reel (32) with a thread (33) whose end can be guided with the sewing needle (35),
- a support (28) for the material to be sewn, with a thread gripper (31) which chain-stitches the thread (33) after the material is pierced through with the needle, and
- a feed device (27) for the material,
**characterized in that**
the device comprises a support tube (23) over which the vascular prosthesis in compressed form can be pushed, for generating a deliberate and permanent curvature on an artificial vascular prosthesis, wherein for generating the curvature at least one longitudinal seam gathers the prosthesis wall while preserving an accordion-like structure and in the compacted state of the pleating in the longitudinal direction.

2. Use according to claim 1, **characterized in that** the external diameter of the support tube (23) corresponds approximately to the internal diameter of the vascular prosthesis (21).

3. Use according to claim 1 or 2, **characterized in that** the support tube (23) is connected to the feed device (27).

4. Use according to any one of the preceding claims, **characterized in that** the support tube (23) has such a high degree of stiffness that, during the sewing procedure, its cross-sectional shape is not changed, or is changed only inappreciably, by the radial loading exerted by the sewing needle (35) on the pushed-on vascular prosthesis (21).

5. Use according to any one of the preceding claims, **characterized in that** the support tube (23) encloses a thread gripper (31).

6. Use according to claim 5, **characterized in that** the thread gripper (31) rotates.

7. Use according to claim 5 or 6, **characterized in that** the diameter of the thread gripper (31) is at most 3 mm smaller than the internal diameter of the support tube (23).

8. Use according to any one of the preceding claims, **characterized in that** the support tube (23) has, in its wall, an oblong hole (36) through which the needle (35) can pass during the sewing procedure.

9. Use according to any one of the preceding claims, **characterized in that** the support tube (23) has, on its outer circumference, at least one axially adjustable abutment (29) for the vascular prosthesis (21).

10. Use according to any one of the preceding claims, **characterized in that** the support tube (23) has, on its outer circumference, at least one further abutment (30) for fixing the compressed vascular prosthesis (21).

## Revendications

1. Utilisation d'un dispositif se composant d'une machine à coudre à point de chaînette avec
- un bras, qui comprend l'entraînement d'aiguille (24) pour une aiguille (35),
- une bobine (32) avec un fil (33), dont l'extrémité peut être guidée avec l'aiguille (35),
- un support (28) pour le matériau à coudre avec un serre-fil (31), qui enchaîne le fil (33) après le piquage à travers le matériau avec l'aiguille, et
- un dispositif d'avance (27) pour le matériau,
**caractérisée en ce que** le dispositif comprend un tube de soutien (23), sur lequel la prothèse vasculaire peut être enfilée sous forme aplatie, pour la production d'une courbure appropriée permanente d'une prothèse vasculaire artificielle, dans lequel pour la production de la courbure au moins une couture longitudinale drape la paroi de prothèse avec obtention d'une structure de type soufflet et dans un état du plissage compacté en direction longitudinale.

2. Utilisation selon la revendication 1, **caractérisée en ce que** le diamètre extérieur du tube de soutien (23) correspond sensiblement au diamètre intérieur de la prothèse vasculaire (21).

3. Utilisation selon la revendication 1 ou 2, **caractérisée en ce que** le tube de soutien (23) est relié au dispositif d'avance (27).

4. Utilisation selon l'une quelconque des revendications précédentes, **caractérisée en ce que** le tube de soutien (23) présente une rigidité telle que la forme de sa section transversale ne change pas ou seulement de façon imperceptible pendant l'opération de couture à cause de la charge radiale appliquée par l'aiguille (35) à la prothèse vasculaire enfilée (21).

5. Utilisation selon l'une quelconque des revendications précédentes, **caractérisée en ce que** le tube de soutien (23) entoure un serre-fil (31).

6. Utilisation selon la revendication 5, **caractérisée en ce que** le serre-fil (31) tourne.

7. Utilisation selon une des revendications 5 ou 6, **caractérisée en ce que** le diamètre du serre-fil (31) est au maximum 3 mm plus petit que le diamètre intérieur du tube de soutien (23).

8. Utilisation selon l'une quelconque des revendications précédentes, **caractérisée en ce que** le tube de soutien (23) présente dans la paroi un trou oblong (36), à travers lequel l'aiguille (35) peut passer pendant l'opération de couture.

9. Utilisation selon l'une quelconque des revendications précédentes, **caractérisée en ce que** le tube de soutien (23) présente sur sa périphérie extérieure au moins une butée réglable axialement (29) pour la prothèse vasculaire (21).

10. Utilisation selon l'une quelconque des revendications précédentes, **caractérisée en ce que** le tube de soutien (23) présente sur sa périphérie extérieure au moins une autre butée (30) pour le serrage de la prothèse vasculaire aplatie (21).
